(19) Europäisches Patentamt
European Patent Office
Office européen des brevets

(11) Publication number: **0 422 466 A1**

# (12) EUROPEAN PATENT APPLICATION

(21) Application number: **90118701.3**

(51) Int. Cl.5: **A61K 7/48**, A61K 7/42

(22) Date of filing: **28.09.90**

(30) Priority: **09.10.89 JP 263747/89**

(43) Date of publication of application:
**17.04.91 Bulletin 91/16**

(84) Designated Contracting States:
**DE FR GB IT**

(71) Applicant: **Sansho Seiyaku Co., Ltd.**
**26-7, Oike 2-chome**
**Onojo-shi Fukuoka-ken(JP)**

(72) Inventor: **Egoshi, Kazuo, No. 105, Riverside Shibuta**
**10-1 Higashiori 3-chome**
**Onojo-shi, Fukuoka-ken(JP)**

(74) Representative: **Patentanwälte Deufel- Schön- Hertel- Lewald**
**Isartorplatz 6**
**W-8000 München 2(DE)**

## (54) Endermic preparation of external application.

(57) Disclosed is an endermic preparation for external application which contains 5-hydroxymaltol or 3-hydroxykojic acid as a skin-whitening or melanogenesis-inhibiting component along with a cosmetic base. The preparation is applied to the human skin to display a noticeable skin-whitening and melanogenesis-inhibiting activity.

## FIG. 1

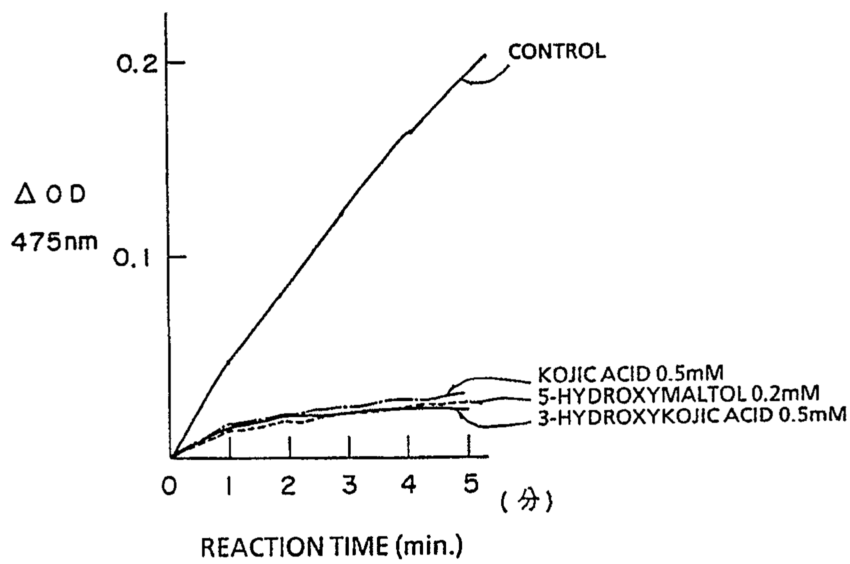


EP 0 422 466 A1

# ENDERMIC PREPARATION FOR EXTERNAL APPLICATION

## BACKGROUND OF THE INVENTION

### Field of the Invention:

The present invention relates to an endermic preparation for external application and, more precisely, to that containing 5-hydroxymaltol or 3-hydroxykojic acid.

### Prior Art:

Hitherto, incorporation of various kinds of peroxides to a cosmetic material to form a skin-whitening cosmetic material has been known. However, known peroxides which are incorporated as a skin-whitening agent, such as hydrogen peroxide, zinc peroxide, magnesium perborate or sodium perborate, are all unstable compounds. Precisely, hydrogen peroxide easily decomposes, and additionally it is in danger of exploding in the presence of a catalyst of a metal colloid, metal oxide or alkali. The other peroxides have a property of easily decomposing under heat or in the presence of water. Therefore, these could not possibly be used as an ingredient to be incorporated into cosmetic materials, though they are admitted to have an excellent characteristic of a sufficient bleaching effect. On the other hand, a skin-whitening cosmetic material containing a bismuth compound or a mercury compound has also been known for a long time. However, the compounds have a problem with respect to the toxicity and harmful side effect to human bodies and, therefore, also they could not be used as an ingredient to be incorporated into cosmetic materials like the above-mentioned peroxides.

Recently, vitamin C, cystein and colloidal sulfur have also been used, but the skin-whitening effect of these substances is extremely weak. Therefore, cosmetic materials containing the substances could not still be said satisfactory skin-whitening cosmetic materials.

The present inventors have continued to investigate and study skin-whitening cosmetic materials, ointments and the like endermic preparations for external application for a long period of time and have invented a skin-whitening cosmetic material and other endermic preparations for external application, containing a skin-whitening component of a kojic acid, a kojic acid derivative or an isokojic acid, which have been illustrated in Japanese Patent Publication (KOKOKU) Nos. 56-18569, 60-9722, 61-60801 and 62-59084.

## SUMMARY OF THE INVENTION

In the course of supplementing the above inventions, the present inventors have further studied and investigated to obtain compounds which are stable and have an excellent melanogenesis-inhibiting effect and, as a result, have found that 5-hydroxymaltol and 3-hydroxykojic acid have an excellent melanogenesis-inhibiting effect. On the basis of such findings, they have succeeded in obtaining excellent endermic preparations for external application.

Specifically, the gist of the present invention resides in an endermic preparation for external application, which contains 5-hydroxymaltol or 3-hydroxykojic acid in an endermic base.

## BRIEF EXPLANATION OF THE DRAWING

Fig. 1 is a graph showing the results of in vitro-tyrosinase activity-inhibiting tests.

## DETAILED DESCRIPTION OF THE INVENTION

5-Hydroxymaltol which is an effective ingredient in the endermic preparation of the present invention is a water-soluble substance as represented by the formula:

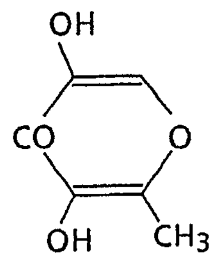

which has a molecular weight of 142 and a melting point of from 185.0 to 186.0° C. 3-Hydroxykojic acid which is another active ingredient in the endermic preparation of the present invention is a water-soluble substance as represented by the formula:

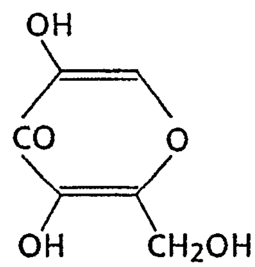

which a molecular weight of 157 and a melting point of from 181.0 to 182.0° C.

5-Hydroxymaltol and 3-hydroxykojic acid which are used in the present invention may be prepared either by incubation of microorganisms or by chemical synthesis.

The endermic preparation for external application of the present invention may be used as a skin-whitening cosmetic material which is in any form of a lotion, cream, an emulsion or a pack and also an endermic medicine which is in any form of ointment or cataplasm. The above-mentioned 5-hydroxymaltol or 3-hydroxykojic acid is incorporated into the bases used in these endermic preparations along with the agents and the like.

The amount of 5-hydroxymaltol or 3-hydroxykojic acid added to the endermic preparation of the invention is from 0.001 to 20.0%, preferably from 0.5 to 10.0%, to the total preparation.

Preferred embodiments of the endermic preparation for external application of the present invention which contains 5-hydroxymaltol or 3-hydroxykojic acid will be explained in detail hereunder.

Lotion:

A moisturizing agent such as glycerin or propylene glycol and a skin nutrient are dissolved in pure water, and the resulting solution is mixed with ethyl alcohol wherein an antiseptic and a perfume have been dissolved at room temperature to prepare a cosmetic base. To the base is added a necessary amount of the above-mentioned 5-hydroxymaltol or 3-hydroxykojic acid.

Cream:

A moisturizing agent such as glycerin or sorbitol is added to pure water to prepare an aqueous phase part. A necessary amount of the above-mentioned 5-hydroxymaltol or 3-hydroxykojic acid is added thereto.

A solid oil component such as bee's wax, paraffin, microcrystalline wax, ceresine, higher fatty acids or solidified oil, a liquid oil component such as squalane, liquid paraffin or various ester oils, and an additional oily component such as an antiseptic or surfactant are mixed to prepare an oily phase part.

Next, the aqueous phase part is gradually heated up to about 80 ° C, to which is added the oily phase part which has also been heated up to almost the same temperature, little by little and thereafter emulsified to obtain cream.

Emulsion:

A moisturizing agent such as glycerin and a pH-adjusting agent such as an acid or alkali are added to pure water and then heated and mixed. A determined amount of 5-hydroxymaltol or 3-hydroxykojic acid is added thereto to prepare an aqueous phase part.

As an oily phase part, an oily component such as an antiseptic or surfactant are added to the mixture of a solid oil component such as bee's wax or paraffin, a semi-solid oil component such as vaseline or lanolin and a liquid oil component such as squalane, liquid paraffin or various ester oils, and then mixed and heated up to about 80 ° C.

Next, the oily phase part is added to the aqueous phase part which has also been heated up to almost the same temperature and pre-emulsified. To this is added a protective colloid agent such as carboxyvinyl polymer or carboxymethyl cellulose and uniformly emulsified with a homomixer to prepare an emulsion.

Pack:

A moisturizing agent such as glycerin and a filming agent such as polyvinyl alcohol or bee gum are added to a pure water and swollen. If necessary, a powder of kaolin, talc or zinc oxide is added thereto. Ethyl alcohol wherein a perfume and an antiseptic have been dissolved is also added thereto, and the mixture is kneaded into a paste. At any stage of the steps, a determined amount of the above-mentioned 5-hydroxymaltol or 3-hydroxykojic acid is added.

Ointment:

A hydrophilic component comprising 5-hydroxymaltol or 3-hydroxykojic acid and a moisturizing agent such as glycerin or sorbitol are added to a pure water to give an aqueous phase part.

On the other hand, an oily phase part is prepared by adding an oily component such as an antiseptic or surfactant along with horse oil to the mixture of a solid oil component such as bee's wax, paraffin, microcrystalline wax, ceresine, higher fatty acids or hardened oil, a semi-solid oil component such as vaseline, lanolin or glyceride and a liquid oil component such as squalane, liquid paraffin or various esters oils. Next, the aqueous phase part is gently stirred while heating up to about 80 ° C, to which the oily phase part which has also been heated up to almost the same temperature is gradually added to obtain an ointment.

As mentioned above, 5-hydroxymaltol or 3-hydroxykojic acid may be incorporated freely into the endermic base in accordance with the present invention. However, where the endermic preparation is composed of an oily phase and an aqueous phase, it is necessary to incorporate the active ingredient of 5-hydroxymaltol or 3-hydroxykojic into the aqueous phase.

Next, the present invention will be explained in more detail by way of the following examples, which, however, are not intended to restrict the scope of the present invention.

Example 1:

| <Cream> | (% by weight) |
|---|---|
| 1. Polyethylene glycol (40E.O>) Monostearate | 2.00 |
| 2. Self-emulsifying Glycerin Monostearate | 5.00 |
| 3. Stearic Acid | 5.00 |
| 4. Behenyl Alcohol | 1.00 |
| 5. Liquid Paraffin | 10.00 |
| 6. Glycerin Trioctanoate | 10.00 |
| 7. 5-Hydroxymaltol | 4.00 |
| 8. Paraoxy-benzoate | 0.20 |
| 9. 1,3-Butylene Glycol | 5.00 |
| 10. Disodium Edetate | 0.01 |
| 11. Pure Water | to make 100 |

Manufacture Method:

A. (1) to (7) were heated and dissolved.
B. (8) to (12) were heated and dissolved.
C. (B) was added to (A) and emulsified, stirred and cooled.
D. (C) was cooled and filled into a container. After testing, the product is sold.

Suggested Use:

An appropriate amount of the product is applied to the face.

Example 2:

| <Emulsion> | (% by weight) |
|---|---|
| 1. Polyethylene (20E.O.) Sorbitol Monostearate | 1.00 |
| 2. Polyoxyethylene (60E.O.) Sorbitol Tetraoleate | 0.50 |
| 3. Oleophilic Glycerin Monostearate | 1.00 |
| 4. Stearic Acid | 0.50 |
| 5. Benhenyl Alcohol | 0.50 |
| 6. Avocado Oil | 4.00 |
| 7. Glyceryl Trioctanoate | 4.00 |
| 8. 3-Hydroxykojic Acid | 3.00 |
| 9. Paraoxy-benzoate | 0.20 |
| 10. 1,3-Butylene Glycol | 5.00 |
| 11. Xanthane Gum | 0.14 |
| 12. Disodium Edetate | 0.01 |
| 13. Pure Water | to make 100 |

Manufacture Method:

A. (1) to (8) were heated and dissolved.
B. (9) to (14) were heated and dissolved.
C. (B) was added to (A) and emulsified, stirred and cooled.
D. (C) was cooled and filled into a container. After testing, the product is sold.

Suggested Use:

An appropriate amount of the product is applied to the face.

Example 3:

| <Lotion> | (% by weight) |
|---|---|
| 1. Polyoxyethylene (60E.O.)-hardened Caster Oil | 8.00 |
| 2. Ethanol | 15.00 |
| 3. 5-Hydroxymaltol | 1.00 |
| 4. Paraoxy-benzoate | 0.10 |
| 5. Citric Acid | 0.10 |
| 6. Disodium Citrate | 0.30 |
| 7. 1,3-Butylene Glycol | 4.00 |
| 8. Disodium Edetate | 0.01 |
| 9. Pure Water | to make 100 |

Manufacture Method:

A. (1) to (10) were uniformly stirred and dissolved.
B. (A) was filled into a container. After testing, the product is sold.

Suggested Use:

An appropriate amount of the product is applied to the face.

Example 4

| <Cream Pack> | (% by weight) |
|---|---|
| 1. Polyethylene Glycol (40E.O.) Monostearate | 2.00 |
| 2. Self-emulsifying Glycerin Monostearate | 5.00 |
| 3. Stearic Acid | 5.00 |
| 4. Behenyl Alcohol | 0.50 |
| 5. Squalane | 15.00 |
| 6. Cetyl Octanoate | 5.00 |
| 7. 3-Hydroxykojic Acid | 5.00 |
| 8. Paraoxy-benzoate | 0.20 |
| 9, 1,3-Butylene Glycol | 5.00 |
| 10. Disodium Edetate | 0.01 |
| 11. Pure Water | to make 100 |

Manufacture Method:

A. (1) to (7) were heated and dissolved.
B. (8) to (11) were heated and dissolved.
C. (B) was added to (A) and emulsified, stirred and cooled.
D. (C) was cooled and then filled into a container.
After testing, the product is sold.

Suggested Use:

An appropriate amount of the product is applied to the face.

Example 5:

| <Ointment> | (% by weight) |
|---|---|
| 1. Sorbitol Monostearate Polyoxyethylene (20E.O.) | 1.00 |
| 2. Polyoxyethylene (60E.O.) Sorbitol Tetraoleate | 1.50 |
| 3. Self-emulsifying Glycerin Monostearate | 1.50 |
| 4. Bleached Bees Wax | 2.00 |
| 5. Paraffin | 2.00 |
| 6. Stearic Acid | 3.00 |
| 7. Behenyl Alcohol | 3.00 |
| 8. Liquid Paraffin | 5.00 |
| 9. Avocado Oil | 12.00 |
| 10. Vitamin E | 0.20 |
| 11. 5-Hydroxymaltol | 10.00 |
| 12. Paraoxy-benzoate | 0.20 |
| 13. 1,3-Butylene Glycol | 5.00 |
| 14. Pure Water | to make 100 |

Manufacture Method:

A. (1) to (11) were heated and dissolved.
B. (12) to (14) were heated and dissolved.
C. (B) was added to (A) and emulsified, stirred and cooled.
D. (C) was cooled and then filled into a container.
After testing, the product is sold.

Suggested Use:

An appropriate amount of the product is applied to the face.

Example 6:

| <Emulsion> | (% by weight) |
|---|---|
| 1. Polyoxyethylene (20E.O.) Sorbitol Monostearate | 1.00 |
| 2. Polyoxyethylene (60E.O.) Sorbitol Tetraoleate | 0.50 |
| 3. Oleophilic Glycerin Monostearate | 1.00 |
| 4. Stearic Acid | 0.50 |
| 5. Behenyl Alcohol | 0.50 |
| 6. Avocado Oil | 4.00 |
| 7. Glyceryl Trioctanoate | 4.00 |
| 8. 3-Hydroxykojic Acid | 3.00 |
| 9. Paraoxy-benzoate | 0.20 |
| 10. 1,3-Butylene Glycol | 5.00 |
| 11. Xanthane Gum | 0.14 |
| 12. Disodium Edetate | 0.10 |
| 13. Pure Water | to make 100 |

Manufacture Method:

A. (1) to (8) were heated and dissolved.
B. A part of (9) to (13) as heated and dissolved.
C. (B) was added to (A) and emulsified, stirred and cooled.
D. (C) was cooled and then filled in a container.
After testing, the product is sold.

Evaluation of Melanogenesis-inhibiting Effect of 5-Hydroxymaltol and 3-Hydroxykojic Acid:

Tyrosinase activity-inhibiting effect and whitening effect in cultured B16 cells of 5-hydroxymaltol and 3-hydroxykojic acid were evaluated in accordance with the following methods.

1. In vitro inhibition of tyrosinase activity:

(1) Preparation of samples:

5-Hydroxymaltol was dissolved in a 0.1M phosphate buffer (pH 6.8) to prepare a sample solution having a final concentration of 0.2 mM.

Separately, 3-hydroxykojic acid was dissolved in a 0.1 M phosphate buffer (pH 6.8) to prepare a sample solution having a final concentration of 0.5 mM.

For comparison, a comparative sample solution containing 0.5 mM kojic acid was prepared and used in the test.

(2) Reaction Condition:

| | |
|---|---|
| Tyrosinase Enzyme Liquid (supernatant of B16 melanoma 30,000G) | 0.1 ml |
| 10 mM Dopa Liquid | 1.0 ml |
| Sample Solution | 2.0 ml |

These were incubated at 37 °C.

(3) Measurement:

Increase of OD at 475 nm was measured at intervals of 30 seconds up to 5 minutes.

(4) Results:

The results are shown in Fig. 1.

From the results, it is noted that the OD increase at 475 nm of the sample containing 0.2 mM 5-hydroxymaltol corresponds to about 12% of the control and that the sample containing 0.2 mM 5-hydroxymaltol has the same inhibiting effect as the comparative sample containing 0.5 mM kojic acid.

The OD increase at 475 nm of the sample containing 0.5 mM 3-hydroxykojic acid corresponds to about 15% of the control and the sample displayed a slightly higher inhibiting effect than the kojic acid-containing sample.

2. Whitening effect in cultured B16 cells:

(1) Preparation of sample-added medium:

5-Hydroxymaltol was dissolved in Eagle's MEM (manufactured by Nissui Pharmaceutical Co.) to prepare a sample-added liquid.

The liquid was added to a medium supplemented with 10% fetal bovine serum in a final concentration of 0.1, 0.5 and 1.0 mM.

3-Hydroxykojic acid was also processed and added to a medium supplemented with 10% fetal bovine serum in a final concentration of 0.5, 1.0, 2.0 and 3.0 mM.

For comparison, kojic acid was also processed and added to a medium supplemented with 10% fetal bovine serum in a final concentration of 2.0 and 3.0 mM and used in the test.

(2) Incubation:

B16 cell suspensions were seeded at $1 \times 10^5$ cells in plastic dishes (Falcon 3003) in Eagle's MEM supplemented with 10% fetal bovine serum and incubated therein for 5 days.

All cultures were maintained in a 5% $CO_2$ humidified air incubator at 37 °C.

The medium was once exchanged 3 days after initiation of incubation.

(3) Preparation of Cell Pellets:

5 days after incubation, cells were peeled from the plastic dishes and suspended in PBS (manufactured by Nissui Pharmaceutical Co.). After centrifugation, cell pellets were prepared.

(4) Results:

Noticeable whitening of the cell pellets was visually observed with the naked eye, in the 0.5 and 1.0 mM 5-hydroxymaltol-added sample.

Precisely, the whitening effect of 5-hydroxymaltol against cultured B16 cells was about from 2 to 3 times of that of kojic acid.

Further, noticeable whitening of the cell pellets was also visually observed with the naked eye in the 2.0 and 3.0 mM 3-hydroxykojic acid-added sample, and the whitening effect of 3-hydroxykojic acid in cultured B16 cells was almost the same as that of kojic acid.

While the invention has been described in detail and with reference to specific embodiments thereof, it

will be apparent to one skilled in the art that various changes and modifications can be made therein without departing from the spirit and scope thereof.

## Claims

1. An endermic preparation for external application, which contains 5-hydroxymaltol or 3-hydroxykojic acid along with a cosmetic base.
2. The endermic preparation for external application as in claim 1, in which the content of 5-hydroxymaltol or 3-hydroxykojic acid is from 0.001 to 20.0% to the total preparation.
3. The endermic preparation for external application as in claim 1 or 2, in which the content of 5-hydroxymaltol or 3-hydroxykojic acid is from 0.5 to 10.0% to the total preparation.

FIG. 1

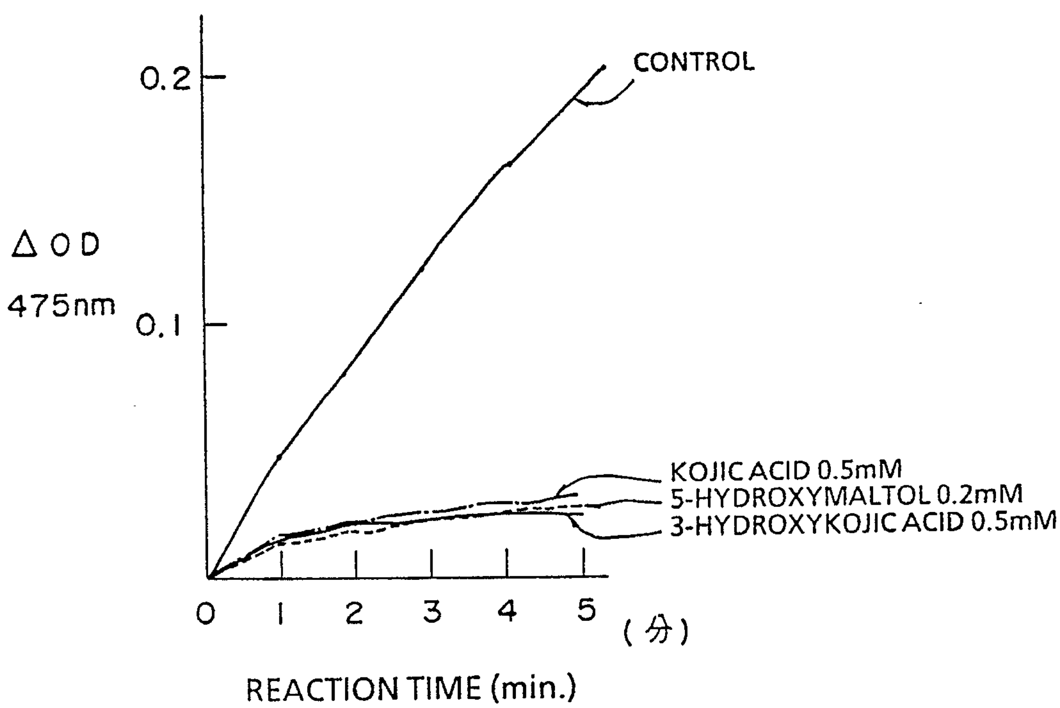
CONTROL
ΔOD
475nm
0.2
0.1
KOJIC ACID 0.5mM
5-HYDROXYMALTOL 0.2mM
3-HYDROXYKOJIC ACID 0.5mM
0
1
2
3
4
5
(分)
REACTION TIME (min.)

European Patent Office

# EUROPEAN SEARCH REPORT

Application Number

EP 90 11 8701

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | S.T.N. FILE SUPPLIER, Karlsruhe, DE, FILE CHEMICAL ABSTRACTS, vol. 106, no. abstract no. 55628y, Columbus, Ohio, US;<br>& JP-A-61 197 506 (YAKURIGAKU CHUO KENKYUSHO K.K.) 01-09-1986<br>- - - | 1-3 | A 61 K<br>7/48<br>A 61 K 7/42 |
| A | CHEMICAL ABSTRACTS, vol. 97, no. 26, December 1982, page 426, abstract no. 222756t, Columbus, Ohio, US;<br>& JP-A-82 134 409 (SANSEI PHARMACEUTICAL CO., LTD) 19-08-1982<br>- - - | 1-3 | |
| A | FR-A-2 613 622 (L. CARIEL et al.)<br>* Tables I,II *<br>- - - - - | 1-3 | |

TECHNICAL FIELDS SEARCHED (Int. Cl.5)

A 61 K

The present search report has been drawn up for all claims

| Place of search | Date of completion of search | Examiner |
|---|---|---|
| The Hague | 18 January 91 | FISCHER J.P. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document